(19) Europäisches Patentamt
European Patent Office
Office européen des brevets

(11) Veröffentlichungsnummer: **0 042 568**
**B1**

# (12) EUROPÄISCHE PATENTSCHRIFT

(45) Veröffentlichungstag der Patentschrift: **04.05.83**

(51) Int. Cl.³: **C 07 C 85/06**, C 07 C 87/50

(21) Anmeldenummer: **81104604.4**

(22) Anmeldetag: **15.06.81**

## (54) Verfahren zur Herstellung von 2,6-Xylidin.

(30) Priorität: **24.06.80 DE 3023487**
**14.03.81 DE 3109986**

(43) Veröffentlichungstag der Anmeldung: **30.12.81 Patentblatt 81/52**

(45) Bekanntmachung des Hinweises auf die Patenterteilung: **04.05.83 Patentblatt 83/18**

(84) Benannte Vertragsstaaten: **BE CH DE GB IT LI**

(56) Entgegenhaltungen:
**DE-B-1 933 636**
**DE-B-2 208 827**
**DE-C-1 289 530**
**US-A-1 935 209**
**US-A-2 013 873**

(73) Patentinhaber: **BAYER AG, Zentralbereich Patente, Marken und Lizenzen, D-5090 Leverkusen 1, Bayerwerk (DE)**

(72) Erfinder: **Böhm, Siegfried, Dr., Meisenweg 7, D-4047 Dormagen (DE)**
Erfinder: **Le Blanc, Helmut, Dr., Am Muehlenteich 5, D-5220 Waldbroel (DE)**
Erfinder: **Wedemeyer, Karlfried, Dr., Bilharzstrasse 7, D-5000 Koeln 80 (DE)**

**EP 0 042 568 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 2,6-Xylidin

Die Erfindung betrifft ein Verfahren zur Herstellung von 2,6-Xylidin durch Aminierung von 2,6-Dimethylphenol in der Dampfphase mit Ammoniak in Gegenwart eines Aluminiumoxidkatalysators.

Es ist bekannt, Phenole in der Dampfphase mit Ammoniak unter Druck an Aluminiumoxid-haltigen Katalysatoren zu den entsprechenden Anilinen umzusetzen (US-PS 1 935 209, US-PS 2 013 873, US-PS 3 272 865, DE-AS 2 026 053, DE-OS 2 003 842). Obwohl in den genannten Druckschriften eine Vielzahl von Phenolen als geeignete Ausgangsverbindungen für die Umsetzung aufgezählt werden, werden in den dort angegebenen Beispielen nur einige wenige Phenole, insbesondere Phenol selbst, m- und p-Kresol und 3,5-Xylenol eingesetzt (US-PS 3 272 865, US-PS 1 935 209, US-PS 2 013 873).

Der DE-OS 2 516 316 zufolge treten bei Aminierungsreaktionen mit substituierten Phenolen Schwierigkeiten auf. So wird beispielsweise erwähnt, daß bei Einsatz von m-Kresol bis zu 10% Nebenprodukte entstehen. Zur Vermeidung dieser Nachteile wird in der DE-OS 2 516 316 der Zusatz von Toluol empfohlen. In den aus dem Stand der Technik bekannten Aminierungsreaktionen sind 2,6-substituierte Phenole überhaupt noch nicht eingesetzt worden. Die Herstellung von 2,6-Xylidin erfolgte bisher durch Umsetzung von Aluminium-tris-(2,6-dimethylphenolat) mit Ammoniak bei einer Temperatur von etwa 200 bis 500° C (DE-AS 1 933 636).

Nach einem anderen Verfahren (DE-AS 2 208 827) setzt man 2,6-Dimethylphenol mit Ammoniak bei 200 bis 400° C in Gegenwart eines Wasserstoffübertragungskatalysators sowie in Gegenwart von Wasser und katalytischen Mengen eines Cyclohexanons zu 2,6-Xylidin um.

Aufgrund der sterischen Hinderung durch die 2,6-Substituenten und der aus DE-OS 2 516 316 bekannten Lehre, daß o-Substituenten die Aminierungsreaktion behindern und zur Bildung von Nebenprodukten Anlaß geben, ist verständlich, daß die an sich bekannte Aminierungsreaktion für die Herstellung von 2,6-Xylidin nicht angewandt wurde und man auf die aufwendigen Verfahren nach der DE-AS 1 933 636 und DE-AS 2 208 827 zur Herstellung von 2,6-Xylidin ausgewichen ist.

Setzt man 2,6-Dimethyl-phenol unter den bekannten Aminierungsbedingungen um, so stellt man auch tatsächlich einen vergleichsweise geringen Umsatz fest, und man beobachtet in erheblichem Ausmaß die Bildung von Nebenprodukten, insbesondere eine Isomerisierung und Disproportionierung unter Bildung von Anilin, o-, m- und p-Toluidin, isomeren Xylidinen und Trimethylanilinen. So liefert die Umsetzung von 2,6-Xylenol mit Ammoniak in der Gasphase unter den in der DE-AS 2 026 053 beschriebenen Aminierungsbedingungen nach 50 Stunden bei 95% Umsatz ein Produktgemisch mit einem 2,6-Xylidin-Gehalt von nur etwa 50%.

Abgesehen von dem durch die schlechte Selektivität bewirkten Ausbeuteverlust bereiten besonders die beträchtlichen Anteile von isomeren 2,4- und 2,5-Xylidinen bei der Aufarbeitung große Schwierigkeiten, da sich vor allem das 2,4-Dimethyl-anilin nicht oder nur mit sehr großem Destillationsaufwand abtrennen läßt.

Auch die Erhöhung des Druckes auf die in der DE-AS 2 026 053 angegebene obere Grenze von 70 bar bringt beim Einsatz eines in der DE-AS 2 026 053 verwendeten Katalysators weder eine Verbesserung des Umsatzes noch der Selektivität mit sich.

Es wurde nun ein Verfahren zur Herstellung von 2,6-Xylidin durch Aminierung von 2,6-Dimethylphenol in der Dampfphase mit Ammoniak in Gegenwart eines Aluminiumoxid-Katalysators gefunden, das dadurch gekennzeichnet ist, daß man die Aminierung bei Temperaturen von 360° C bis 460° C und bei Drücken von 70—250 bar bei einem Mindest-Molverhältnis von Ammoniak zu 2,6-Dimethylphenol durchführt, das der allgemeinen Formel

$$X = e^{\left(\frac{Y-B}{M}\right)},$$

worin

| | |
|---|---|
| X | das Mindest-Molverhältnis von Ammoniak zu 2,6-Dimethylphenol wiedergibt, |
| Y | für den Mindest-Druck in bar steht und |
| M und B | Konstanten mit $M = -39{,}2826$ und $B = 262{,}809$ sind, |

entspricht.

Das erfindungsgemäße Verfahren wird bei einem Molverhältnis von Ammoniak zu 2,6-Dimethylphenol von mindestens etwa 1,4 : 1 und einem Mindest-Druck von etwa 70 bar durchgeführt. Nach oben bestehen sowohl beim Ammoniak/2,6-Dimethylphenol-Molverhältnis als auch beim Druck lediglich technische und wirtschaftliche Grenzen. Bei erhöhtem Druck muß allerdings beachtet werden, daß das Ammoniak/2,6-Dimethylphenol-Gemisch im Reaktor noch dampfförmig und nicht schon flüssig vorliegt. Im allgemeinen wird das erfindungsgemäße Verfahren bei einem Molverhältnis von Ammoniak zu 2,6-Dimethylphenol von mindestens 3 : 1, bevorzugt von mindestens 5 : 1 und bei Drücken von 70 bis 250, bevorzugt von 90 bis 220, bar durchgeführt.

Es empfiehlt sich dabei in technischen Anlagen, die Aminierung bei hohen Drücken durchzuführen, damit nur kleine Ammoniak-Mengen durchgesetzt werden müssen und auf diese Weise eine aufwendige Ammoniak-Wiederaufbereitung vermieden werden kann.

Gemäß der oben angegebenen Formel erhält man z. B. für ein gewähltes Ammoniak/2,6-Dimethylphenol-Molverhältnis von 15 : 1, 30 : 1, 70 : 1 oder 75 : 1 einen korrespondierenden Mindestdruck von 156, 129, 96 bzw. 93 bar, der bei den vorgegebenen Ammoniakmengen erforderlich ist, um den Gehalt an isomeren Xylidinen bei vollständigem 2,6-Dimethylphenol-Umsatz unter 1% zu halten.

Das erfindungsgemäße Verfahren wird üblicherweise bei Temperaturen von 360 bis 460°C, bevorzugt bei 380 bis 440°C, besonders bevorzugt bei 400 bis 430°C, durchgeführt.

Als Katalysatoren haben sich Aluminiumoxidkatalysatoren bewährt, die mindestens 95 Gew.-% Aluminiumoxid, weniger als 0,5% Natriumoxid und weniger als 0,5% Eisenoxid enthalten. Bevorzugt wird ein hochreiner Aluminiumoxid-Katalysator eingesetzt, der höchstens 0,2 Gew.-% Alkalimetall (angegeben als Alkalimetalloxid) und höchstens 0,3 Gew.-% Eisenmetall (angegeben als Eisenoxid) enthält. Insbesondere werden ein hochreiner Aluminiumoxidkatalysator der BASF mit der Typenbezeichnung D 10-10 (Firmenschrift der BASF: BASF-Katalysatoren, K 0020 d, e, f 2.74, Seite 27) oder die Aluminiumkatalysatoren der Rhône Progil mit den Typenbezeichnungen SAS 350, SCS 250 und SCS 100 (Firmenschrift der Rhône Progil: Alumina Catalyst Carries SPHERALITE 09.73.10) bevorzugt.

Die einzusetzenden Aluminiumoxidkatalysatoren können in Form von Preßlingen oder Kugeln verwendet werden, bevorzugt werden Preßlinge mit einem Durchmesser von etwa 1,5 mm bis etwa 10 mm eingesetzt.

Das erfindungsgemäße Verfahren kann beispielsweise wie folgt durchgeführt werden:

Das 2,6-Dimethylphenol wid mit einem Überschuß an Ammoniak durch das mit dem Aluminiumoxidkatalysator gefüllte Reaktionsrohr unter Druck bei höheren Temperaturen geleitet. Nach dem Reaktionsrohr wird das Reaktionsgemisch entspannt und kondensiert. Das Kondensat wird in an sich bekannter Weise fraktioniert destilliert. Zur Rückführung des nicht umgesetzten Ammoniakanteils kann der Ammoniak bei ca. 50 bar von dem Produktgemisch abdestilliert und im Kreis gefahren werden.

In einer bevorzugten Ausführungsform des erfindungsgemäßen Verfahrens setzt man bei der Umsetzung Wasser hinzu. Im allgemeinen ist es zweckmäßig bis zu 15 Gew.-% Wasser, bezogen auf die Menge des eingesetzten Ammoniaks und des 2,6-Dimethylphenols, hinzuzusetzen. Bevorzugt gibt man 5 bis 10 Gew.-% Wasser hinzu. Durch diese bevorzugte Arbeitsweise kann die Selektivität der Reaktion noch verbessert werden.

Das erfindungsgemäße Verfahren kann sowohl diskontinuierlich als auch kontinuierlich durchgeführt werden.

Überraschenderweise ist es mit Hilfe des erfindungsgemäßen Verfahrens möglich, 2,6-Xylidin praktisch frei von den störenden isomeren 2,4- und 2,5-Xylidinen herzustellen, so daß die Isolierung des 2,6-Xylidins in über 99%iger Reinheit ohne Schwierigkeiten gewährleistet ist.

2,6-Xylidin ist ein Zwischenprodukt zur Herstellung von Herbiziden (vgl. DE-OS 2 648 008, DE-OS 2 305 495, US-PS 3 952 056 und 4 019 894). Die Herbizide können beispielsweise durch Acylierung und Veretherung des 2,6-Xylidins hergestellt werden.

Das erfindungsgemäße Verfahren sei anhand der nachfolgenden Beispiele erläutert.

A) Aluminiumoxid-Katalysator

1. $\gamma$-$Al_2O_3$-Preßling mit einem Durchmesser von 10 bis 15 mm
Oberfläche: 230 $m^2/g$

Zusammensetzung des trockenen Katalysators:

| | |
|---|---|
| $Al_2O_3$ | 99% |
| $Na_2O$ | 0,2% |
| $Fe_2O_3$ | 0,3% |

2. $\gamma$-$Al_2O_3$-Kugeln mit einem Durchmesser von 2 bis 5 mm
Oberfläche: 350 $m^2/g$

Zusammensetzung des Katalysators:

| | |
|---|---|
| $Al_2O_3$ | 99% |
| $Na_2O$ | 0,7% |
| $Fe_2O_3$ | 0,025% |
| $SiO_2$ | 0,02% |

3. $\gamma$-$Al_2O_3$-Kugeln mit einem Durchmesser von 4 bis 6 mm
Oberfläche: 275 $m^2/g$

Zusammensetzung des Katalysators:

| | |
|---|---|
| $Al_2O_3$ | 99% |
| $Na_2O$ | 0,08% |
| $Fe_2O_3$ | 0,025% |
| $SiO_2$ | 0,02% |

4. $\gamma$-$Al_2O_3$-Kugeln mit einem Durchmesser von 2 bis 4 mm
Oberfläche: 80 bis 100 $m^2/g$

Zusammensetzung des Katalysators:

| | |
|---|---|
| $Al_2O_3$ | 99% |
| $Na_2O$ | 0,08% |
| $Fe_2O_3$ | 0,025% |
| $SiO_2$ | 0,02% |

5. $\gamma$-$Al_2O_3$-Kugeln mit einem Durchmesser von 2 bis 3 mm
Oberfläche: 390 $m^2/g$

Zusammensetzung des trockenen Katalysators:

| | |
|---|---|
| $Al_2O_3$ | 96,5% |
| $Na_2O$ | 1,5% |
| $Fe_2O_3$ | 0,1% |
| $SiO_2$ | 1,8% |

B) Herstellung von 2,6-Xylidin

Beispiel 1

Ein Reaktorrohr wird mit 880 ml $\gamma$-$Al_2O_3$-Preßlingen der Zusammensetzung von A1 beschickt und auf 400° C aufgeheizt. Durch Stahlkapillaren, die ebenfalls bei 400° C gehalten werden, pumpt man 2,6-Dimethyl-phenol mit einer Geschwindigkeit von 37,5 g (=0,31 Mol) Xylenol in der Stunde und Ammoniak mit einer Geschwindigkeit von 313,4 g (=18,4 Mol) $NH_3$ in der Stunde in den heißen Reaktor, wobei mit Hilfe eines Druckhalteventils im Reaktor ein Druck von 190 bar eingestellt wird.

Das durch das Ventil austretende Gasgemisch wird kondensiert und in einem Vorlagegefäß gesammelt; um Ammoniak im Kreis zu fahren, destilliert man das überschüssige $NH_3$ bei abgesenktem Druck (ca. 50 bar) vom Produktgemisch ab, kondensiert und führt das verflüssigte Ammoniak in die Reaktion zurück.

Man erhält in der Stunde 34,1 g 2,6-Xylidin.

Nach 50 h enthält das vom Reaktionswasser abgetrennte Produktgemisch:

| | |
|---|---|
| 90,8% | 2,6-Dimethyl-anilin, |
| 0,1% | 2,4-Dimethyl-anilin, |
| 0,2% | 2,5-Dimethyl-anilin, |
| 0,2% | 2,6-Dimethyl-phenol und |
| 8,2% | Anilin sowie einfach oder mehrfach methylsubstituierte Aniline. |

Beispiel 2

Die Arbeitsweise von Beispiel 1 wird mit der Änderung wiederholt, daß durch

| | |
|---|---|
| 880 ml | Katalysator der Zusammensetzung von A1 |
| 20,1 g | 2,6-Dimethyl-phenol (0,16 Mol) in der Stunde und |
| 125,9 g | Ammoniak (7,4 Mol) in der Stunde |

gepumpt werden. Bei einer Temperatur von 400° C wird im Reaktor ein Druck von 190 bar gehalten.

Man erhält in der Stunde 18,8 g 2,6-Xylidin.

Das nach 50 Stunden entnommene Produktgemisch setzt sich aus

| | |
|---|---|
| 93,6% | 2,6-Dimethyl-anilin, |
| 0,1% | 2,4-Dimethyl-anilin, |
| 0,1% | 2,5-Dimethyl-anilin, |
| 0,2% | 2,6-Dimethyl-phenol und |
| 5,5% | Anilin sowie einfach oder mehrfach methylsubstituierten Anilinen zusammen. |

Beispiel 3

Die Arbeitsweise von Beispiel 1 wird mit der Änderung wiederholt, daß durch 880 ml Katalysator der Zusammensetzung von A1 17,5 g 2,6-Dimethyl-phenol (0,14 Mol) in der Stunde und 146,4 g Ammoniak (8,6 Mol) in der Stunde gepumpt werden. Bei einer Temperatur von 400° C wird im Reaktor ein Druck von 160 bar gehalten.

Man erhält in der Stunde 15,8 g 2,6-Dimethylanilin.

Das nach 50 Stunden entnommene Produktgemisch setzt sich aus

| | |
|---|---|
| 90,0% | 2,6-Dimethyl-anilin |
| 0,2% | 2,4-Dimethyl-anilin |
| 0,2% | 2,5-Dimethyl-anilin |
| 0,2% | 2,6-Dimethyl-phenol und |
| 8,9% | Anilin sowie einfach oder mehrfach methylsubstituierten Anilinen zusammen. |

Beispiel 4

Die Arbeitsweise von Beispiel 1 wird mit der Änderung wiederholt, daß durch 880 ml Katalysator der Zusammensetzung von A1 24,4 g 2,6-Dimethyl-phenol (0,2 Mol) in der Stunde und 305,7 g $NH_3$ (18,0 Mol) in der Stunde gepumpt werden. Bei einer Temperatur von 400° C wird im Reaktor ein Druck von 130 bar gehalten.

Man erhält in der Stunde 21,7 g 2,6-Xylidin.

Das nach 50 Stunden entnommene Produktgemisch setzt sich aus

| | |
|---|---|
| 89,1% | 2,6-Dimethyl-anilin, |
| 0,3% | 2,4-Dimethyl-anilin, |
| 0,3% | 2,5-Dimethyl-anilin, |
| 0,3% | 2,6-Dimethyl-phenol und |
| 9,3% | Anilin sowie einfach oder mehrfach methylsubstituierten Anilinen zusammen. |

Beispiel 5

Die Arbeitsweise von Beispiel 1 wird mit der Änderung wiederholt, daß durch 880 ml Katalysator der Zusammensetzung von A2 29,8 g (0,24 Mol) 2,6-Dimethyl-phenol in der Stunde und 249,2 g (14,7 Mol) $NH_3$ in der Stunde gepumpt werden. Bei einer Temperatur von 400° C wird im Reaktor ein Druck von 190 bar gehalten.

Man erhält in einer Stunde 21,5 g 2,6-Xylidin.

Das nach 50 Stunden entnommene Produktgemisch setzt sich aus

| | |
|---|---|
| 72,0% | 2,6-Dimethyl-anilin, |
| 0,7% | 2,4-Dimethyl-anilin, |
| 0,2% | 2,5-Dimethyl-anilin, |
| 11,6% | 2,6-Dimethyl-phenol und |
| 13,2% | Anilin sowie einfach oder mehrfach methylsubstituierten Anilinen zusammen. |

Beispiel 6

Die Arbeitsweise von Beispiel 1 wird mit der Änderung wiederholt, daß durch 880 ml Katalysator der Zusammensetzung von A3 19,0 g (0,16 Mol) 2,6-Dimethyl-phenol in der Stunde und 159,3 g (9,4 Mol) $NH_3$ in der Stunde gepumpt werden. Bei einer Temperatur von 400° C wird im Reaktor ein Druck von 190 bar gehalten.

Man erhält in einer Stunde 14,8 g 2,6-Xylidin.

Das nach 50 Stunden entnommene Produktgemisch setzt sich aus

77,8% 2,6-Dimethyl-anilin,
0,5% 2,4-Dimethyl-anilin,
0,1% 2,5-Dimethyl-anilin,
9,0% 2,6-Dimethyl-phenol und
11,2% Anilin sowie einfach oder mehrfach methylsubstituierten Anilinen zusammen.

Beispiel 7

Die Arbeitsweise von Beispiel 1 wird mit der Änderung wiederholt, daß durch 880 ml Katalysator der Zusammensetzung von A4 10,1 g (0,08 Mol) 2,6-Dimethyl-phenol in der Stunde und 84,8 g (5,0 Mol) $NH_3$ in der Stunde gepumpt werden. Bei einer Temperatur von 400° C wird im Reaktor ein Druck von 190 bar gehalten.

Man erhält in einer Stunde 8,6 g 2,6-Xylidin.

Das nach 50 Stunden entnommene Produktgemisch setzt sich aus

84,5% 2,6-Dimethyl-anilin,
0,5% 2,4-Dimethyl-anilin,
0,2% 2,5-Dimethyl-anilin,
0,7% 2,6-Dimethyl-phenol und
13,5% Anilin sowie einfach oder mehrfach methylsubstituierten Anilinen zusammen.

Beispiel 8

Ein Reaktorrohr wird mit 1200 ml $\gamma$-$Al_2O_3$-Preßlingen der Zusammensetzung von A1 beschickt und auf 400° C aufgeheizt.

Man pumpt eine Lösung von 732 g (6 Mol) 2,6-Dimethyl-phenol in 6120 g (360 Mol) Ammoniak und 686 g (38,1 Mol) Wasser aus einem Vorlagegefäß über ein Steigrohr durch eine auf 400° C beheizte Kapillare mit einer Geschwindigkeit von 377 g (22,2 Mol) $NH_3$ bzw. 45,1 g (0,37 Mol) Xylenol in der Stunde in den heißen Reaktor, wobei mit Hilfe eines Druckhalteventils im Reaktor ein Druck von 190 bar eingestellt wird.

Das durch ein Ventil austretende Gasgemisch wird kondensiert und in einem Vorlagegefäß gesammelt; zur Rückgewinnung des überschüssigen Ammoniaks destilliert man das nicht umgesetzte $NH_3$ bei abgesenktem Druck (ca. 50 bar) vom Produktgemisch ab.

Man erhält in der Stunde 41,7 g 2,6-Xylidin. Das nach 50 Stunden entnommene Produktgemisch setzt sich aus

92,3% 2,6-Dimethyl-anilin,
0,1% 2,4-Dimethyl-anilin,
0,1% 2,5-Dimethyl-anilin,
1,8% 2,6-Dimethyl-phenol und
5,6% Anilin sowie einfach oder mehrfach methylsubstituierten Anilinen zusammen.

C) Vergleichsbeispiele nach DE-AS 2 026 053

Beispiel 9

Die Arbeitsweise von Beispiel 1 wird mit der Änderung wiederholt, daß durch 880 ml Katalysator der Zusammensetzung von A1 10,6 g (0,09 Mol) 2,6-Dimethyl-phenol in der Stunde und 29,5 g (1,7 Mol) $NH_3$ in der Stunde gepumpt werden ($NH_3$/Xylenol = 20 : 1).

Bei einer Temperatur von 380° C wird im Reaktor ein Druck von 17 bar gehalten.

Man erhält in einer Stunde 5,3 g 2,6-Xylidin.

Das nach 50 Stunden entnommene Produktgemisch setzt sich aus

49,7% 2,6-Dimethyl-anilin,
6,0% 2,4-Dimethyl-anilin,
3,4% 2,5-Dimethyl-anilin,
4,2% 2,6-Dimethyl-phenol und
32,7% Anilin sowie einfach oder mehrfach methylsubstituierten Anilinen zusammen.

Beispiel 10

Die Arbeitsweise von Beispiel 1 wird mit der Änderung wiederholt, daß durch 880 ml Katalysator der Zusammensetzung von A5 7,8 g (0,06 Mol) 2,6-Dimethyl-phenol in der Stunde und 21,8 g (1,3 Mol) $NH_3$ in der Stunde gepumpt werden ($NH_3$/Xylenol = 20 : 1).

Bei einer Temperatur von 380° C wird in dem Reaktor ein Druck von 70 bar gehalten.

Man erhält in einer Stunde 3,1 g 2,6-Xylidin.

Das nach 50 Stunden entnommene Produktgemisch setzt sich aus

39,4% 2,6-Dimethyl-anilin,
11,1% 2,4-Dimethyl-anilin,
0,5% 2,5-Dimethyl-anilin,
3,2% 2,6-Dimethyl-phenol und
42,7% Anilin sowie einfach oder mehrfach methylsubstituierten Anilinen zusammen.

**Patentansprüche**

1. Verfahren zur Herstellung von 2,6-Xylidin durch Aminierung von 2,6-Dimethylphenol in der Dampfphase mit Ammoniak in Gegenwart eines Aluminiumoxid-Katalysators, dadurch gekennzeichnet, daß man die Aminierung bei Temperaturen von 360 bis 460° C und bei Drücken von

70—250 bar bei einem Mindest-Molverhältnis von Ammoniak zu 2,6-Dimethylphenol durchführt, das der allgemeinen Formel

$$X = e^{\left(\frac{Y-B}{M}\right)},$$

worin

X das Mindest-Molverhältnis von Ammoniak zu 2,6-Dimethylphenol wiedergibt,
Y für den Mindest-Druck in bar steht und
M und B Konstanten mit M = −39,2826 und B = 262,809 sind,

entspricht.

2. Verfahren nach Anspruch 1, dadurch gekennzeichnet, daß man die Aminierung bei Temperaturen von 380 bis 440° C durchführt.

3. Verfahren nach Anspruch 1, dadurch gekennzeichnet, daß man die Aminierung bei Temperaturen von 400 bis 430° C durchführt.

4. Verfahren nach Ansprüchen 1 bis 3, dadurch gekennzeichnet, daß man die Aminierung mit einem Molverhältnis von Ammoniak zu 2,6-Dimethylphenol von mindestens 3 : 1 durchführt.

5. Verfahren nach Ansprüchen 1 bis 3, dadurch gekennzeichnet, daß man die Aminierung bei Molverhältnissen von Ammoniak zu 2,6-Dimethylphenol von mindestens 5 : 1 durchführt.

6. Verfahren nach Ansprüchen 1 bis 5, dadurch gekennzeichnet, daß man die Aminierung bei Drücken von 90—220 bar durchführt.

7. Verfahren nach Ansprüchen 1 bis 6, dadurch gekennzeichnet, daß man die Aminierung in Gegenwart von bis zu 15 Gew.-% Wasser, bezogen auf die Menge des eingesetzten Ammoniaks und des 2,6-Dimethylphenols, durchführt.

8. Verfahren nach Ansprüchen 1 bis 6, dadurch gekennzeichnet, daß man die Aminierung in Gegenwart von 5—10 Gew.-% Wasser, bezogen auf die Menge des eingesetzten Ammoniaks und des 2,6-Dimethylphenols, durchführt.

## Claims

1. Process for the preparation of 2,6-xylidine by amination of 2,6-dimethylphenol in the vapour phase with ammonia in the presence of an aluminium oxide catalyst, characterised in that the amination is carried out at temperatures from 360 to 460° C and under pressures of 70—250 bars at a minimum molar ratio of ammonia to 2,6-dimethylphenol which corresponds to the general formula

$$X = e^{\left(\frac{Y-B}{M}\right)},$$

wherein

X represents the minimum molar ratio of ammonia to 2,6-dimethylphenol,
Y represents the minimum pressure in bars and
M and B are constants, with M = −39.2826 and B = 262.809.

2. Process according to claim 1, characterised in that the amination is carried out at temperatures from 380 to 440° C.

3. Process according to claim 1, characterised in that the amination is carried out at temperatures from 400 to 430° C.

4. Process according to claims 1 to 3, characterised in that the amination is carried out with a molar ratio of ammonia to 2,6-dimethylphenol of at least 3 : 1.

5. Process according to claims 1 to 3, characterised in that the amination is carried out at molar ratios of ammonia to 2,6-dimethylphenol of at least 5 : 1.

6. Process according to claims 1 to 5, characterised in that the amination is carried out under pressures of 90—220 bars.

7. Process according to claims 1 to 6, characterised in that the amination is carried out in the presence of up to 15% by weight of water, relative to the amount of ammonia employed and to the amount of 2,6-dimethylphenol.

8. Process according to claims 1 to 6, characterised in that the amination is carried out in the presence of 5—10% by weight of water, relative to the amount of ammonia employed and to the amount of 2,6-dimethylphenol.

## Revendications

1. Procédé de préparation de 2,6-xylidine par amination de 2,6-diméthylphénol en phase vapeur avec de l'ammoniac en présence d'un catalyseur d'oxyde d'aluminium, caractérisé en ce qu'on effectue l'amination à des températures de 360 à 460° C et à des pressions de 70 à 250 bars avec un rapport molaire minimal entre l'ammoniac et le 2,6-diméthylphénol, correspondant à la formule générale

$$X = e^{\left(\frac{Y-B}{M}\right)},$$

dans lequel

X représente le rapport molaire minimal entre l'ammoniac et le 2,6-diméthylphénol
Y représente la pression minimale en bar et
M et B sont des constantes telles que M = −39,2826 et B = 262,809

2. Procédé selon la revendication 1, caractérisé en ce qu'on effectue l'amination à des températures de 380 à 440° C.

3. Procédé selon la revendication 1, caractérisé en ce qu'on effectue l'amination à des températures de 400 à 430° C.

4. Procédé selon les revendications 1 à 3, caractérisé en ce qu'on effectue l'amination avec un rapport molaire entre l'ammoniac et le 2,6-diméthylphénol d'au moins 3 : 1.

5. Procédé selon les revendications 1 à 3, caractérisé en ce qu'on effectue l'amination avec des rapports molaires entre l'ammoniac et le 2,6-diméthylphénol d'au moins 5 : 1.

6. Procédé selon les revendications 1 à 5, caractérisé en ce qu'on effectue l'amination à des pressions de 90 à 220 bars.

7. Procédé selon les revendications 1 à 6, caractérisé en ce qu'on effectue l'amination en présence de jusqu'à 15% en poids d'eau par rapport à la quantité de l'ammoniac mis en oeuvre et du 2,6-diméthylphénol.

8. Procédé selon les revendications 1 à 6, caractérisé en ce qu'on effectue l'amination en présence de 5 à 10% en poids d'eau par rapport à la quantité d'ammoniac mis en oeuvre et du 2,6-diméthylphénol.